Europäisches Patentamt

(19) European Patent Office

Office européen des brevets

(11) Numéro de publication : **0 513 176 B1**

(12) **FASCICULE DE BREVET EUROPEEN**

(45) Date de publication du fascicule du brevet :
**26.01.94 Bulletin 94/04**

(51) Int. Cl.⁵ : **A61M 1/10, A61M 1/12**

(21) Numéro de dépôt : **91904121.0**

(22) Date de dépôt : **08.02.91**

(86) Numéro de dépôt international :
**PCT/FR91/00098**

(87) Numéro de publication internationale :
**WO 91/12035 22.08.91 Gazette 91/19**

(54) **PROCEDE ET DISPOSITIF DE REGULATION DE DEBIT D'UNE PROTHESE CARDIAQUE A DEBIT PERIODIQUE.**

(30) Priorité : **09.02.90 FR 9001850**
**09.02.90 FR 9001851**

(43) Date de publication de la demande :
**19.11.92 Bulletin 92/47**

(45) Mention de la délivrance du brevet :
**26.01.94 Bulletin 94/04**

(84) Etats contractants désignés :
**DE ES FR GB IT**

(56) Documents cités :
**WO-A-85/04812**
**WO-A-88/03784**
**DE-A- 2 355 966**
**FR-A- 2 458 288**
**US-A- 3 911 897**
**US-A- 3 919 722**
**IEEE Transactions on Biomedical Engineering, volume 37, No. 2, February 1990, IEEE, (New York, US), W.J. Weiss et al.: "Permanent circulatory support systems at the Pennsylvania State University", pages 138 - 144 see page 138, right-hand column, line 1 - page 139, left-hand column, line 43; figure 3**
**IEEE Engineering in Medicine and Biology, volume 5, No. 1, March 1986, IEEE, (New York, US), D. J. Farrar et al. : "Control modes of a clinical ventricular assist device", pages 19 - 25 see page 20, lines 11 - 55; figure 2**
**IEEE Proceedings of the fifteenth Annual Northeast Bioengineering Conference, Boston, MA, 27 - 28 March 1989, IEEE, (New York, US), M.A. Ignatoski et al.: "An aortic pressure observer for the penn state electric ventricular assist device - A deterministic design", pages 79 - 80**

(56) Documents cités :
**IEEE Proceedings of the Seventh Annual Conference of the IEEE/Engineering in Medicine and Biology Society, Chicago, Illinois, 27-30 September 1985, volume 2, No. CH 2198-0/85, IEEE, (New York, US), A.L. Oslan et al.: "Non-percutaneous monitoring of a permanently implanted ventricular assist system", pages 819 - 823 see page 819, left-hand column, line 1 - right-hand column, line 26; figure 1**
**L'Onde Electrique, volume 67, No. 1, January 1987, (Paris, FR), P. Havlik et al.: "Coeur artificiel implantable entrainé par actionneur électrique asservi", pages 87-98 see page 91, figure 3; page 94, column 1, lines 42-56; page 95, column 1, lines 23-26**

(73) Titulaire : **TERACOR**
**80 Avenue Louis-Lagrange**
**F-83000 Toulon (FR)**

(72) Inventeur : **CANDELON, Bernard, Les Terrasses du Cap-Brun**
**3076, avenue de la Résistance**
**F-83000 Toulon (FR)**
Inventeur : **TRINKL, Jean, La Rouvière**
**Bâtiment B11, 83, boulevard du Redon**
**F-13009 Marseille (FR)**
Inventeur : **HAVLIK, Patrick**
**2, rue Maurin**
**F-13009 Marseille (FR)**
Inventeur : **MONTIES, Jean-Raoul**
**18, Les Hélianthes**
**F-13390 Auriol (FR)**

(74) Mandataire : **Moretti, René et al**
**c/o Cabinet BEAU DE LOMENIE**
**"Prado-Mermoz" 232, Avenue du Prado**
**F-13008 Marseille (FR)**

## Description

La présente invention a pour objet un procédé et un dispositif de régulation de débit d'une pompe cardiaque à débit périodique.

On utilise de plus en plus des prothèses d'assistance ou de remplacement cardiaque pour seconder ou remplacer un coeur humain défaillant ; dans le cas d'assistance on utilise fréquemment une prothèse qui seconde un demi coeur, le gauche ou le droit, alors que pour un remplacement, on utilise une prothèse qui est constituée par deux demi-coeurs artificiels. Ces prothèses comportent toujours au moins une pompe, un système électrique ou pneumatique d'entraînement de la pompe et des moyens de doser l'énergie fournie au système d'entraînement pour assurer un fonctionnement approprié de la prothèse.

Le type de pompe utilisé peut être volumétrique comme les pompes à piston rotatif connues sous les noms de moteur WANKEL ou de pompe CORA décrite dans ce brevet FR 2 389 382, les pompes à membrane, les pompes à piston, ou centrifuges.

Dans la suite du document, on utilisera indifféremment les mots pompe et prothèse pour désigner le dispositif mécanique qui aspire et refoule le sang du patient.

Généralement, ces pompes assurent un débit périodique ou pulsé.

Dans la suite du document sauf indication contraire, le terme période désigne l'intervalle de temps séparant deux passages successifs du débit sanguin véhiculé par la pompe, par la même valeur, avec le même sens de variation.

Généralement cette période de variation du débit sanguin véhiculé par la pompe est égal à, ou est une fraction de la période du mouvement de rotation d'un rotor pour les pompes rotatives ou du mouvement de translation d'un piston pour les pompes à piston ou à membrane.

Les pompes sont souvent raccordées en parallèle sur un coeur ou un demi-coeur qui continue à fonctionner ; le raccordement aux sites d'implantation se fait par l'intermédiaire de canules.

L'entrée ou admission de la pompe est ainsi raccordée au retour veineux pulmonaire ou systémique, parfois au niveau de l'oreillette du coeur du patient, parfois au niveau du ventricule du coeur du patient.

Le débit d'une prothèse ne peut dépasser le débit du sang disponible dans ce retour veineux ; dans le cas contraire, on arriverait à un collapsus des vaisseaux ou a une déformation de la paroi vasculaire ou cardiaque formant clapet, phénomènes qui doivent être absolument évités.

On connaît des dispositifs qui utilisent des capteurs de pression pour contrôler le remplissage correct du site d'implantation dans lequel la prothèse aspire, par comparaison entre la pression mesurée et une pression de référence.

Le brevet FR 2 470 593 (sur lequel se fonde le préambule des revendications 1 à 9) décrit en effet un dispositif régulateur pour prothèse cardiaque qui comporte plusieurs asservissements à la pression sanguine, par comparaison à une pression de référence.

Ces dispositifs mesurent une pression moyenne, et sont sensibles aux variations de paramètres physiques qui peuvent entraîner des variations de la grandeur mesurée sans être significatifs d'un dysfonctionnement de la prothèse ; les paramètres qui influent sur ces mesures sont notamment la pression environnante ou atmosphérique, les accélérations dues aux chocs, aux transports, aux mouvements...

En effet, les pressions dans les différentes cavités cardiaques sont relatives ; la référence est la pression intrathoracique qui est elle-même influencée par l'environnement physique.

De plus, l'amplitude des variations de pression à contrôler est faible par rapport à la grandeur mesurée et nécessite des étalonnages fréquents et coûteux.

Par exemple, l'amplitude des pulsations de la pression auriculaire du coeur droit est comprise entre zéro et 4 millibars environ, entre zéro et 10 millibars environ pour le coeur gauche.

On sait par ailleurs que le rythme cardiaque d'un patient évolue au cours du temps, notamment au cours d'une journée en fonction du métabolisme du patient.

Par conséquent, la vitesse d'entraînement d'une prothèse cardiaque doit pouvoir s'adapter aux évolutions lentes du rythme cardiaque correspondant à ces variations du métabolisme du patient.

A ce jour, ces variations ne sont pas contrôlées par les dispositif de régulation, l'adaptation de la vitesse d'entraînement de la prothèse étant faite manuellement et de manière empirique par la midification d'une consigne de vitesse d'entraînement.

Le problème à résoudre est de procurer des procédés et des dispositifs de régulation fiables pour adapter le débit véhiculé par les prothèses cardiaques assurant un débit périodique, au débit sanguin de remplissage du site d'implantation, très peu sensibles aux influences des paramètres physiques de l'environnement tels que pression atmosphérique, accélération, et ne nécessitant pas des étalonnages fréquents.

Le problème a résoudre est également de procurer un procédé et un dispositif de régulation de prothèse cardiaque qui puisse adapter la vitesse d'entraînement de la prothèse aux variations du métabolisme du patient.

Une solution au problème posé consiste à procurer un procédé de régulation d'une prothèse cardiaque qui comporte un pompe volumétrique assurant un débit périodique de période T et qui comporte un actionneur électrique d'entraînement de ladite pompe, lequel actionneur est alimenté par des moyens d'alimentation électrique, qui comporte les opérations suivantes :

a - on procure au moins un capteur de mesure d'un paramètre physique de fonctionnement de ladite prothèse, dont la bande passante s'étend au-delà de la fréquence F correspondant à ladite période T et on procure des moyens de détermination de la position de ladite prothèse,

b - on détermine au moins une suite de valeurs normales dudit paramètre physique de fonctionnement de ladite prothèse et une suite de positions de ladite prothèse correspondant respectivement auxdites valeurs normales dudit paramètre physique, laquelle suite de valeurs normales dudit paramètre physique doit être observée pendant ladite période T,

c - on détermine au moins un seuil S,

puis lors du fonctionnement de ladite prothèse, pendant chacune desdites périodes, à des intervalles de temps $T_4$ de valeur inférieure à ladite période T, et de préférence à des intervalles de temps $T_4$ régulièrement espacés et tels que le rapport $T/T_4$ soit supérieur à 10,

d - on capte, grâce audit capteur dudit paramètre physique de fonctionnement, des valeurs dudit paramètre physique de fonctionnement et simultanément on détermine, grâce auxdits moyens de détermination de la position, la position de ladite prothèse,

e - on calcule l'écart entre ladite valeur captée dudit paramètre physique de fonctionnement et la valeur normale dudit paramètre physique, de ladite suite de valeurs normales, correspondant à ladite position de ladite prothèse déterminée par lesdits moyens de détermination de la position de ladite prothèse,

f - si ledit écart est supérieur audit seuil, on agit sur lesdits moyens d'alimentation pour réduire la vitesse d'entraînement de ladite pompe cardiaque.

Ledit capteur de mesure d'un paramètre physique de fonctionnement de ladite prothèse peut être un capteur de mesure d'un paramètre de l'écoulement sanguin provoqué par ladite prothèse, par exemple le débit ou la pression sanguine, peut être alternativement un capteur de mesure d'un paramètre mécanique de fonctionnement de ladite prothèse, par exemple la force exercée par ladite pompe sur le sang véhiculé par ladite prothèse ou bien le couple d'entraînement de ladite pompe par ledit actionneur, ou bien alternativement un capteur de mesure d'un paramètre électrique de fonctionnement dudit actionneur électrique d'entraînement de ladite prothèse.

Avantageusement, lorsqu'on modifie la vitesse d'entraînement de ladite pompe, par exemple selon ladite opération f, on initialise des moyens de temporisation de durée $T_1$ tels que le rapport $T_1/T$ soit inférieur à 500 et de préférence inférieur à 50, et lorsque ladite durée $T_1$ est écoulée, on agit sur lesdits moyens d'alimentation pour augmenter la vitesse d'entraînement de ladite pompe cardiaque.

Ainsi, dans un mode préférentiel d'utilisation d'un procédé selon l'invention, lorsqu'on a, selon ladite opération f, fait varier ladite vitesse d'entraînement à partir d'une première valeur, jusqu'à une deuxième valeur inférieure à ladite première valeur, on initialise lesdits moyens de temporisation ; lorsque ladite durée desdits moyens de temporisation est écoulée, on fait varier ladite vitesse d'entraînement à partir de ladite deuxième valeur jusqu'à ladite première valeur, cette augmentation de ladite vitesse d'entraînement pouvant être effectuée progressivement ou brusquement.

Avantageusement, à des intervalles de temps $T_2$ tels que le rapport $T_2/T$ soit supérieur à 1000, on agit sur lesdits moyens d'alimentation pour augmenter, de préférence progressivement et régulièrement, la vitesse d'entraînement de ladite pompe cardiaque jusqu'à ce que ledit écart selon ladite opération f soit supérieur audit seuil, de sorte que l'on détermine -on mesure- ainsi une vitesse maximale d'entraînement de ladite pompe qui correspond à une assistance cardiaque totale d'un malade.

Avantageusement, on procure en outre des moyens de réglage d'une consigne de pourcentage d'assistance d'un malade, et consécutivement à la détection de ladite vitesse maximale d'entraînement de ladite pompe correspondant à une assistance totale, on réduit ladite vitesse d'entraînement de ladite pompe à une valeur sensiblement égale au produit de ladite vitesse maximale par ledit pourcentage d'assistance.

Dans un premier mode préférentiel de mise en oeuvre d'un procédé selon l'invention, ledit capteur de mesure d'un paramètre physique de fonctionnement est un capteur de la composante fluctuante de la pression d'un fluide intervenant dans le fonctionnement de ladite prothèse, et de préférence ledit capteur est un capteur de la composante fluctuante de la pression sanguine à l'entrée de ladite pompe, lequel capteur est très peu sensible aux accélérations, et préalablement à ladite opération b,

- on capte lesdites positions de ladite pompe et simultanément on capte les amplitudes de la composante fluctuante de ladite pression dudit fluide, pendant une période T au moins,

- on calcule des valeurs normales de la composante fluctuante de ladite pression dudit fluide correspondant auxdites positions captées, lesquelles valeurs normales sont très proches desdites amplitudes de la composante fluctuante de ladite pression dudit fluide, et sont situées sur une courbe qui est la représentation graphique d'une fonction constituée par une somme de fonctions trigonométriques dont les périodes sont des fractions de ladite période T,

et de préférence ladite pompe cardiaque est une pompe cardiaque rotative et lesdites positions sont les position angulaires d'un rotor de ladite pompe et/ou du moteur rotatif constituant ledit actionneur, et ladite suite de valeurs normales de la composante fluctuante de ladite pression dudit fluide ainsi que ladite valeur dudit seuil, sont enregistrés dans au moins une mémoire.

Alternativement, ledit fluide intervenant dans le fonctionnement de ladite prothèse peut être un fluide, de préférence sensiblement incompressible, qui est utilisé, notamment dans le cas d'une pompe cardiaque à membrane, comme moyen de transmission ou d'entraînement de ladite membrane.

Dans un deuxième mode préférentiel de mise en oeuvre d'un procédé selon l'invention, ledit capteur de mesure d'un paramètre physique de fonctionnement est un capteur d'un paramètre électrique du fonctionnement dudit actionneur, de préférence un capteur de l'intensité absorbée par ledit actionneur et, dans ladite opération d, en début de ladite période, et pendant une durée $T_5$ inférieure à la moitié de ladite période,

- on utilise lesdites valeurs captées dudit paramètre électrique pour déterminer les conditions de fonctionnement de ladite pompe cardiaque,
- on prédétermine lesdites valeurs normales dudit paramètre électrique qui doivent être observées dans la période courante, après ledit début de ladite période de ladite durée $T_5$, en fonction desdites conditions de fonctionnement et desdites valeurs captées dudit paramètre électrique.

Avantageusement,
- on calcule ledit écart comme étant la somme quadratique des écarts entre lesdites valeurs captées dudit paramètre électrique depuis le début de ladite période et lesdites valeurs normales dudit paramètre électrique,
- on donne une valeur nulle au début de chaque période audit écart et audit seuil,
- on donne audit seuil une valeur qui varie linéairement en fonction du temps écoulé depuis le début de ladite période.

La solution du problème posé consiste également à procurer une pompe volumétrique assurant un débit périodique de période T et qui comporte un actionneur électrique d'entraînement de ladite pompe, lequel actionneur est alimenté par des moyens d'alimentation électrique, tel qu'il comporte en outre au moins un capteur de mesure d'un paramètre physique de fonctionnement de ladite prothèse dont la bande passante s'étend au-delà de la fréquence F correspondant à ladite période T, et ledit dispositif comprend des moyens de détermination de la position de ladite prothèse, au moins une mémoire dans laquelle peuvent être stockées des valeurs normales dudit paramètre physique, au moins une unité centrale qui reçoit des signaux issus dudit capteur d'un paramètre physique et desdits moyens de détermination de la position de ladite prothèse, laquelle unité centrale peut commander lesdits moyens d'alimentation pour faire varier la vitesse dudit actionneur et/ou de ladite pompe.

Avantageusement, ladite pompe est une pompe rotative et ledit actionneur est un moteur synchrone à aimants permanents, et lesdits moyens de détection de la position de ladite prothèse sont des moyens de sonde à effet Hall qui sont sensibles au champ électromagnétique rayonné par ledit moteur, et ladite pompe est raccordée par son entrée à un site d'implantation et par sa sortie au réseau artériel, par des canules, et ladite bande passante s'étend au-delà de 10 fois et de préférence au-delà de 100 fois ladite fréquence F.

Dans un premier mode de réalisation d'un dispositif selon l'invention, ledit capteur d'un paramètre physique de fonctionnement est un capteur de pression sanguine qui est situé entre ledit site d'implantation et ladite entrée de ladite pompe, lequel capteur est très peu sensible aux accélérations, et des valeurs normales de la composante fluctuante de la pression sanguine à l'entrée de ladite pompe sont stockées dans ladite mémoire.

Dans un deuxième mode de réalisation d'un dispositif selon l'invention, ledit capteur d'un paramètre physique de fonctionnement est un capteur d'un paramètre électrique dudit actionneur, de préférence un capteur de l'intensité absorbée par ledit actionneur.

Avantageusement, un dispositif selon l'invention comporte en outre des moyens de réglage d'une consigne de pourcentage d'assistance d'un malade.

Le terme de composante fluctuante utilisé dans ce document se réfère à la terminologie habituellement employée suivante : une grandeur notée G, variable dans le temps, peut être décomposée en une composante moyenne notée $G_1$, et une composante fluctuante notée $G_2$ ; ce qui se traduit par l'équation :

$$G = G_1 + G_2$$

dans laquelle $G_1$ est la moyenne temporelle de G.

L'invention a pour résultat un procédé et un dispositif de régulation de débit d'une pompe cardiaque à débit périodique qui ne sont pas sensibles aux variations des paramètres physiques de l'environnement tels que pression atmosphérique, accélération, et ne nécessitent pas d'étalonnages fréquents.

Un avantage supplémentaire procuré notamment par ledit deuxième mode de réalisation de dispositif est qu'il peut éviter l'emploi de capteur de pression sanguine implanté, ce qui peut éviter les interventions nécessaires en cas de défaut de ce type de capteur implanté.

Les nombreux avantages procurés par l'invention seront mieux compris au travers de la description suivante qui se réfère aux dessins annexés qui représentent, sans aucun caractère limitatif, des exemples de dispositifs et de procédés selon l'invention.

La figure 1 est un schéma représentant un premier mode de réalisation d'un dispositif de régulation selon l'invention.

La figure 2 est un graphe représentant un exemple de la forme des pulsations du débit sanguin aspiré par une pompe cardiaque volumétrique.

La figure 3 est un graphe représentant les principales opérations d'une première phase d'un premier procédé selon l'invention.

La figure 4 est un graphe représentant les principales opérations d'une deuxième phase d'un premier procédé selon l'invention.

La figure 5 est un schéma représentant un deuxième mode de réalisation d'un dispositif de régulation selon l'invention.

La figure 6 est un graphe représentant l'intensité absorbée pendant une période par le moteur électrique d'une pompe cardiaque.

La figure 7 comprend trois graphes 7A, 7B, 7C.

Le graphe 7A représente la pression sanguine à l'entrée d'une pompe cardiaque pendant 5 tours ou périodes.

Le graphe 7B représente la pression sanguine à la sortie d'une pompe cardiaque pendant cinq tours ou périodes.

Le graphe 7c représente l'intensité absorbée par le moteur électrique de cette pompe cardiaque.

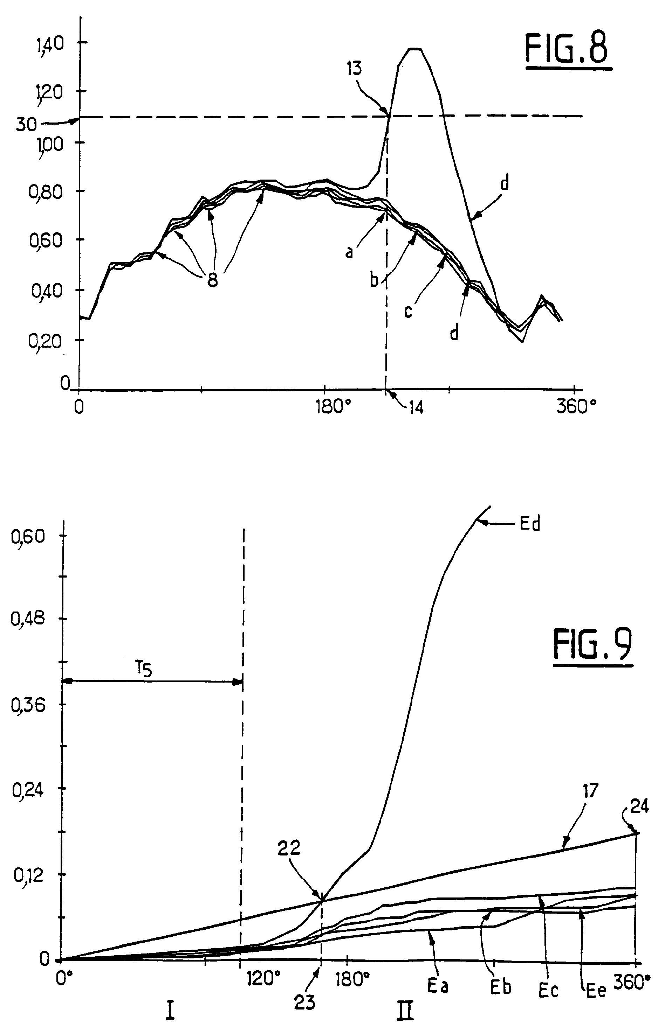

La figure 8 montre l'intensité absorbée par ce moteur pendant ces cinq périodes qui sont superposées.

La figure 9 montre pour ces cinq périodes les écarts entre l'intensité réellement abordée par le moteur et l'intensité normale prévue.

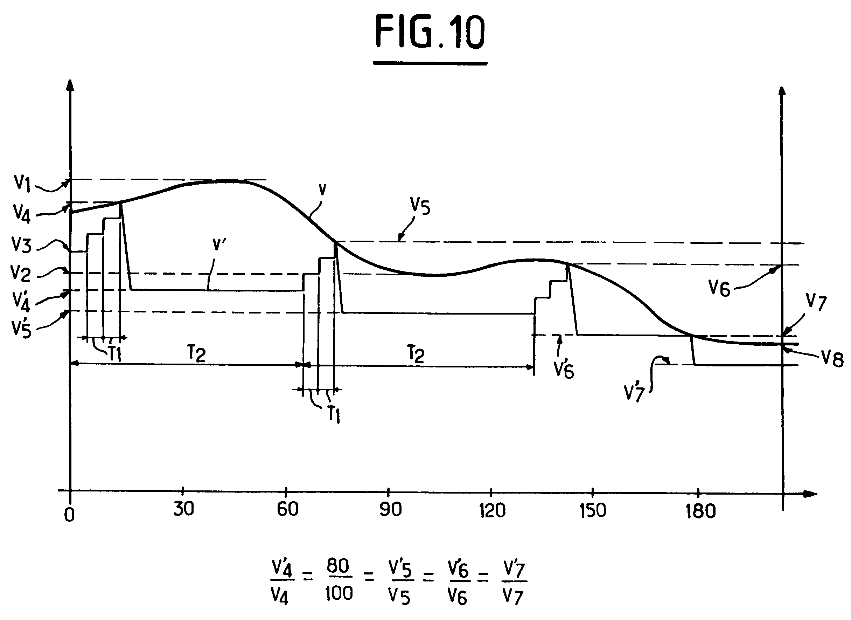

La figure 10 illustre des opérations d'un procédé selon l'invention.

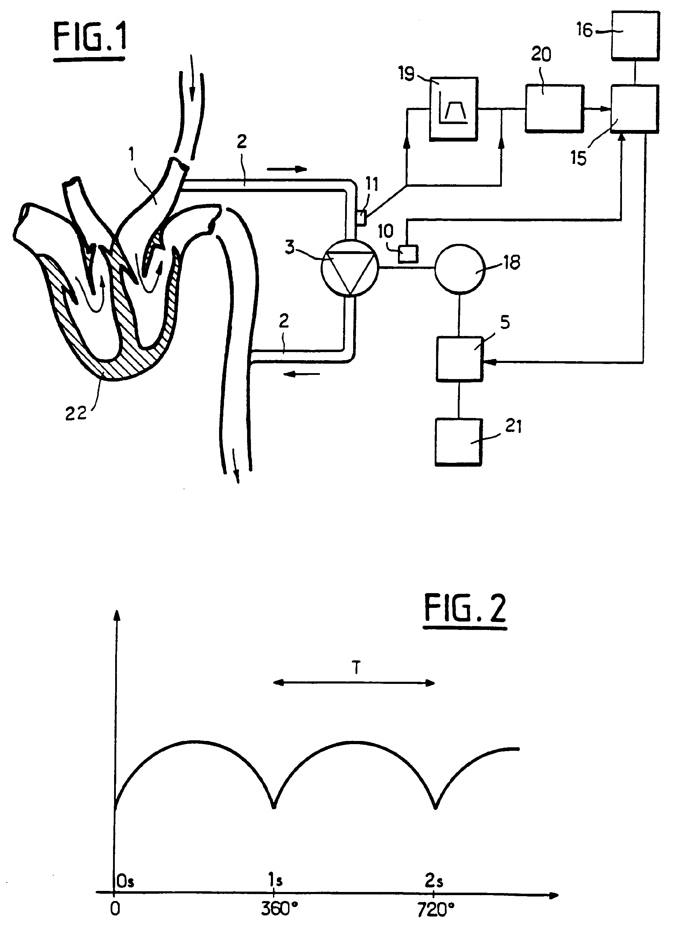

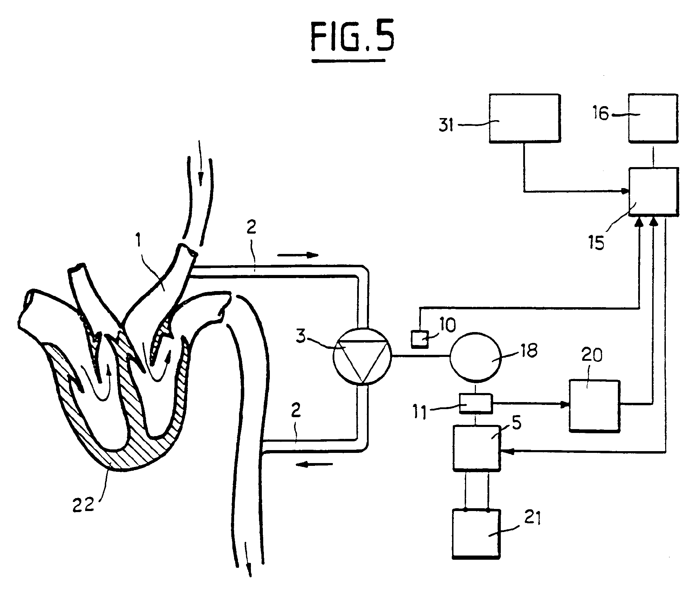

Sur les figures 1 et 5, on a représenté une prothèse cardiaque constituée par une pompe rotative 3 qui assure un débit périodique de période T.

Généralement, la période T a une valeur proche de la période moyenne de battement naturel du coeur ; elle est par exemple comprise entre 250 millisecondes et 2 secondes.

La pompe 3 aspire dans une canule 2 qui est reliée au système sanguin du patient au niveau d'un site d'implantation 1. Le site d'implantation 1 peut se trouver sur le retour veineux ou directement sur l'oreillette du coeur 22.

Ladite pompe 3 refoule dans une autre canule 2 qui communique avec le réseau artériel en aval dudit coeur 22.

Ladite pompe 3 telle que représentée sur les figures 1 et 5 peut constituer une prothèse d'assistance d'une moitié dudit coeur 22 qui peut continuer à fonctionner, mais elle pourrait également constituer une prothèse de remplacement.

Ladite pompe 3 est entraînée en rotation par un moteur 18 qui est alimenté par des moyens d'alimentation 5 qui sont eux-mêmes raccordés à une source d'énergie 21.

Ledit moteur 18 peut être par exemple un moteur électrique sans balais, à aimants permanents, très compact, de telle sorte que l'ensemble constitué par la pompe et son moteur peut être implanté sur un patient.

Lesdits moyens d'alimentation 5 transforment l'énergie disponible dans ladite source 21, pour fournir au moteur une énergie qui lui assure un fonctionnement correct; lesdits moyens d'alimentation sont constitués par exemple par un onduleur statique polyphasé.

Le dispositif selon l'invention peut comprendre un capteur de rotation 10 qui fournit un signal correspondant à la position angulaire d'un rotor de la prothèse, lequel signal est transmis à l'unité centrale 15.

Ledit capteur de position angulaire 10 peut être du type résolver, syncho-transmetteur, codeur absolu, relatif ou incrémental.

Alternativement, un circuit électronique, tel que celui proposé par la Société SGS sous la référence L6230 ou celui proposé par la Société Philips Component sous la référence TOA514X, peut être utilisé, qui permet de simuler lesdites sondes à effet Hall et qui permet ainsi de connaître la position angulaire du rotor dudit moteur électrique d'entraînement de ladite prothèse.

De façon préférentielle, ce capteur a une grande résolution qui peut être supérieure à 1000 points par tour, et peut être également utilisé pour l'asservissement du moteur 18, néanmoins une résolution de 48 points par tour environ s'avère suffisante dans de nombreux cas.

Cette résolution peut être obtenue notamment, grâce à trois sondes à effet Hall placées dans le rotor d'un moteur électrique équipé d'un rotor à aimants permanents, muni de 16 pôles.

Selon un premier mode de réalisation représenté à la figure 1, le dispositif selon l'invention comprend un capteur de pression sanguine 11, qui est un capteur de pression dynamique dont la bande passante s'étend au-delà de la fréquence correspondant à la période T des pulsations de ladite pompe.

Ce capteur est en contact avec le flux sanguin aspiré par la prothèse et est situé entre le site d'implantation 1 et l'aspiration de ladite pompe 3.

Avantageusement, ce capteur est situé sur ladite canule 2, à l'entrée de ladite pompe 3.

Avantageusement, ce capteur de pression est compensé en accélération, afin de diminuer les perturbations du signal qu'il délivre dues aux chocs. Par exemple, ledit capteur de pression est du type piézoélectrique.

Dans ce mode de réalisation d'un dispositif de régulation selon l'invention, ledit capteur de pression 11 est relié à ladite unité centrale 15 par des moyens d'amplification et de conversion 20 qui transforment le signal électrique délivré par ledit capteur de pression 11 de telle sorte qu'il soit compatible avec l'interface de ladite unité centrale 15.

Avantageusement, le signal issu dudit capteur de pression 11 transite par un ou plusieurs filtres 19 qui éliminent les composantes de très haute et de très basse fréquence, lesquelles composantes ne sont pas utiles au dispositif de régulation et peuvent dans certains cas constituer une gêne pour cette régulation de débit.

Ladite unité centrale peut être par exemple une carte électronique pilotée par un micro-processeur. Cette unité centrale est reliée d'une part à une mémoire électronique 16 qui contient les valeurs normales de la composante fluctuante de la pression sanguine à l'entrée de la pompe cardiaque, et d'autre part auxdits moyens d'alimentation 5 qu'elle commande afin de faire varier la vitesse de ladite pompe cardiaque.

Avantageusement, ladite mémoire 16 peut contenir différentes séries de valeurs normales de la composante fluctuante de la pression d'entrée, chaque série correspondant à un type d'environnement différent, par exemple à des valeurs différentes de ladite période T.

Ladite mémoire peut également contenir les valeurs des écarts E admissibles entre les valeurs normales contenues dans ladite mémoire et les valeurs captées de la composante fluctuante de la pression sanguine à l'entrée de ladite prothèse cardiaque.

Ledit capteur de pression 11, ledit filtre 19, lesdits moyens de conversion 20, ladite unité centrale 15, ladite mémoire 16, lesdits moyens d'alimentation 5, coopèrent de telle sorte que lors d'un défaut de remplissage de la cavité cardiaque où est implantée ladite canule 2 reliée à l'entrée de ladite pompe 3, ce défaut entraînant une variation rapide de la pression à l'entrée de la pompe, ladite variation ou fluctuation de pression 8 est captée par ledit capteur de pression 11, est transmise auxdits moyens de conversion 20 après filtrage éventuel par ledit filtre 19, est ensuite transformée par lesdits moyens 20 pour être compatible avec l'interface de ladite unité centrale 15, qui compare ladite variation de pression captée avec la valeur normale 9 stockée dans la mémoire 16 et pilote lesdits moyens d'alimentation 5 afin de diminuer la vitesse de rotation de ladite prothèse et de diminuer ainsi le débit véhiculé par elle, dans le cas où l'écart entre ladite variation 8 et ladite valeur normale 9 est supérieur à un seuil enregistré dans ladite mémoire 16, de telle sorte qu'on évite le collapsus des vaisseaux ou la déformation des parois de la cavité cardiaque.

Sur la figure 2 est représenté un exemple de l'allure des pulsations du débit aspiré par une pompe cardiaque à piston rotatif.

L'axe des abscisses représente le temps ou la position angulaire d'un rotor de la prothèse, quand celui-ci est entraîné à vitesse constante.

L'axe des ordonnées représente le débit véhiculé par ladite prothèse.

La courbe de la figure 2 représente un cas de fonctionnement normal de ladite prothèse cardiaque.

Dans cet exemple la période T vaut sensiblement 1 seconde.

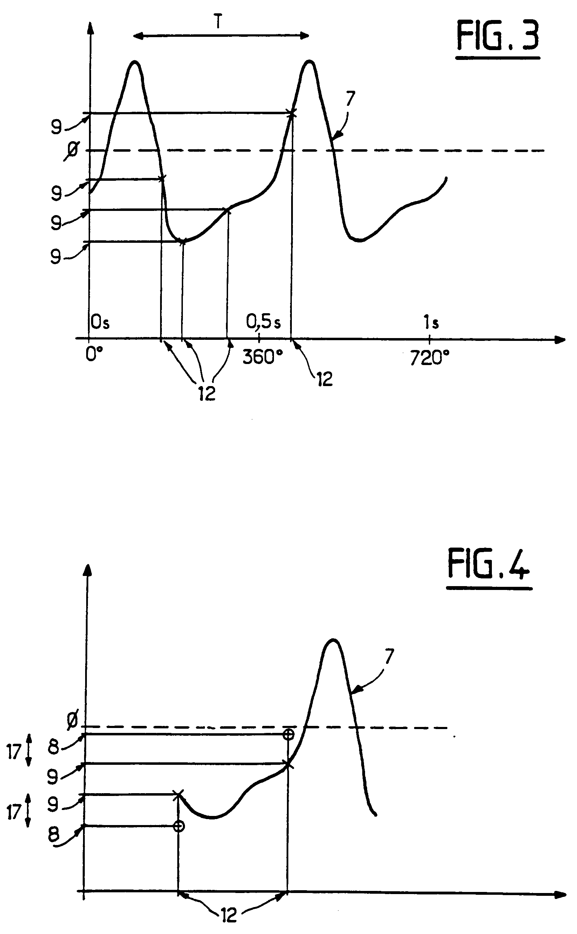

Sur la figure 3 sont représentées sur un graphe les principales opérations effectuées dans la première phase d'un procédé selon l'invention, dite phase préparatoire.

L'axe des abscisses représente le temps ou la position angulaire d'un rotor de la prothèse cardiaque quand celui-ci est entraîné à vitesse constante ; l'axe est gradué en secondes pour le temps et en degrés pour la position angulaire.

L'axe des ordonnées représente la pression sanguine dans la canule 2, à l'endroit où est situé ledit capteur de pression.

La période T de variation du débit sanguin véhiculé par la pompe cardiaque vaut approximativement une demi-seconde.

On a enregistré sur le graphe, pendant une période T, les positions angulaires 12 d'un rotor de ladite prothèse cardiaque et les valeurs normales 9 de la composante fluctuante de la pression d'entrée de ladite pompe cardiaque, chaque couple de valeurs (9, 12) définissant un point sur le graphe.

On a construit une courbe 7 qui passe par les points définis par lesdites positions angulaires 12 et lesdites valeurs normales 9 de la composante fluctuante de la pression sanguine à l'entrée de ladite pompe cardiaque.

Avantageusement, préalablement à l'enregistrement des valeurs normales 9, on capte les positions angulaires 12 et les amplitudes 8 de la composante fluctuante de la pression d'entrée de la pompe pendant une période T au moins, en s'assurant que le fonctionnement de la prothèse est satisfaisant.

On utilise lesdites amplitudes captées 8 pour calculer des valeurs normales 9 de la composante fluctuante de la pression sanguine à l'entrée de la pompe, correspondant auxdites positions angulaires 12, de telle sorte que lesdites valeurs normales 9 soient proches desdites valeurs captées 8.

Avantageusement, on calcule lesdites valeurs normales 9 de sorte qu'elles sont placées sur une courbe 7 qui est la représentation graphique d'une fonction exprimant les variations de la composante fluctuante de la pression d'entrée de la pompe selon la position angulaire d'un rotor de la pompe, laquelle fonction est constituée par une somme de fonctions trigonométriques dont les périodes sont des fractions de ladite période T de variation du débit de la pompe.

Avantageusement, lesdites positions angulaires 12 sont choisies de sorte qu'elles sont régulièrement espacées entre 0 et 360 degrés.

Dans un mode opératoire préférentiel du procédé selon l'invention on enregistre préalablement à la première phase, les valeurs 13 de la composante fluctuante du débit normal véhiculé par la prothèse et on calcule les valeurs normales 9 de la composante fluctuante de la pression à l'entrée de la pompe à partir desdites valeurs 13.

Sur la figure 4 sont représentées sur un graphe les principales opérations effectuées dans la deuxième phase d'un procédé selon l'invention, dite phase de surveillance.

Les axes représentent les mêmes paramètres qu'énoncé pour la figure 3.

On a capté simultanément et reporté sur le graphe ladite position angulaire 12 dudit rotor et ladite amplitude 8 de la composante fluctuante de la pression à l'entrée de ladite pompe.

On a tracé sur ce graphe ladite courbe 7 obtenue dans la phase préparatoire.

On calcule la différence 17 entre ladite amplitude captée 8 de la composante fluctuante de la pression d'entrée et ladite valeur normale 9 de la composante fluctuante de la pression d'entrée, laquelle valeur 9 est l'ordonnée du point de ladite courbe 7 dont l'abscisse correspond à la position angulaire captée 12.

Si la valeur absolue de ladite différence 17 est supérieure audit seuil enregistré dans la phase préparatoire, on réduit la vitesse de rotation de la pompe cardiaque de sorte qu'on réduit son débit.

Avantageusement, après avoir capté ladite amplitude 8 de la composante fluctuante de la pression d'entrée de la pompe, on élimine les composantes de basse et haute fréquence, avant d'effectuer ladite comparaison.

Dans un mode opératoire particulier, on élimine les composantes dont la période est inférieure à la moitié de la période T de variation du débit sanguin véhiculé par la pompe et celles dont la période est supérieure à cinq fois ladite période T.

Dans le cas d'une prothèse cardiaque comprenant une pompe à piston ou à membrane, ledit capteur de position angulaire pourra être remplacé par un capteur de position linéaire.

Selon un deuxième mode de réalisation représenté à la figure 5, un dispositif selon l'invention comprend un capteur d'intensité 11 dont la bande passante s'étend au-delà de la fréquence correspondant à ladite période T des pulsations de débit de la pompe cardiaque.

Ce capteur mesure l'intensité absorbée par ledit moteur 18 ; il peut être de type résistif ou bien être constitué par au moins une sonde à effet Hall, par exemple.

Avantageusement, ce capteur est situé sur, ou inséré dans, le circuit électrique qui relie ledit moteur 18 auxdits moyens d'alimentation 5, au plus près dudit moteur.

Dans ce mode de réalisation d'un dispositif de régulation selon l'invention, ledit capteur d'intensité 11 est relié à l'unité centrale 15 par des moyens d'amplification et de conversion 20 qui transforment le signal électrique délivré par ledit capteur d'intensité 11 de telle sorte qu'il soit compatible avec l'interface de ladite unité centrale 15.

L'unité centrale peut être par exemple une carte électronique pilotée par un micro-processeur. Cette unité centrale est relié d'une part à une mémoire électronique 16 qui contient les valeurs normales de l'intensité absorbée par le moteur 18 de la pompe cardiaque, et d'autre part aux moyens d'alimentation 5 qu'elle commande afin de faire varier la vitesse de la pompe cardiaque.

Avantageusement, des moyens 31 de réglage d'une consigne de pourcentage d'assistance sont également prévus, qui peuvent être reliés audit micro processeur 15, de sorte que lorsqu'on a mesuré une vitesse d'entraînement correspondant à une assistance totale d'un patient, on peut définir une consigne de vitesse d'entraînement correspondant au pourcentage d'assistance cardiaque défini par l'autorité médicale, lequel pourcentage peut être introduit et enregistré dans un dispositif selon l'invention, grâce auxdits moyens de réglage d'une consigne de pourcentage d'assistance.

Avantageusement, ladite mémoire 16 peut contenir différentes séries de valeurs normales de l'intensité, chaque série correspondant à des conditions d'environnement différentes.

Ladite mémoire peut également contenir les valeurs des écarts E admissibles entre lesdites valeurs normales contenues dans la mémoire et les valeurs captées de l'intensité absorbée par ledit moteur 18.

Dans un dispositif selon l'invention, ledit capteur d'intensité 11, lesdits moyens de conversion 20, ladite unité centrale 15, ladite mémoire 16, lesdits moyens d'alimentation 5, coopèrent de telle sorte que lors d'un défaut de remplissage de la cavité cardiaque où est implantée la canule 2 reliée à l'entrée de ladite pompe 3, ce défaut entraînant une variation rapide de l'intensité absorbée par ledit moteur 18, ladite variation d'intensité 8 est captée par ledit capteur d'intensité 11, est transmise auxdits moyens de conversion 20, est ensuite transformée par lesdits moyens 20 pour être compatible avec l'interface de ladite unité centrale 15, qui compare ladite variation d'intensité captée avec ladite valeur normale 9 stockée dans ladite mémoire 16 et pilote lesdits moyens d'alimentation 5 afin de diminuer la vitesse de rotation de ladite prothèse et de diminuer ainsi le débit véhiculé par elle, dans le cas où l'écart entre ladite intensité 8 et ladite valeur normale 9 est supérieur à un seuil S enregistré dans ladite mémoire 16, de telle sorte qu'on évite le collapsus des vaisseaux ou la déformation des parois de la cavité cardiaque.

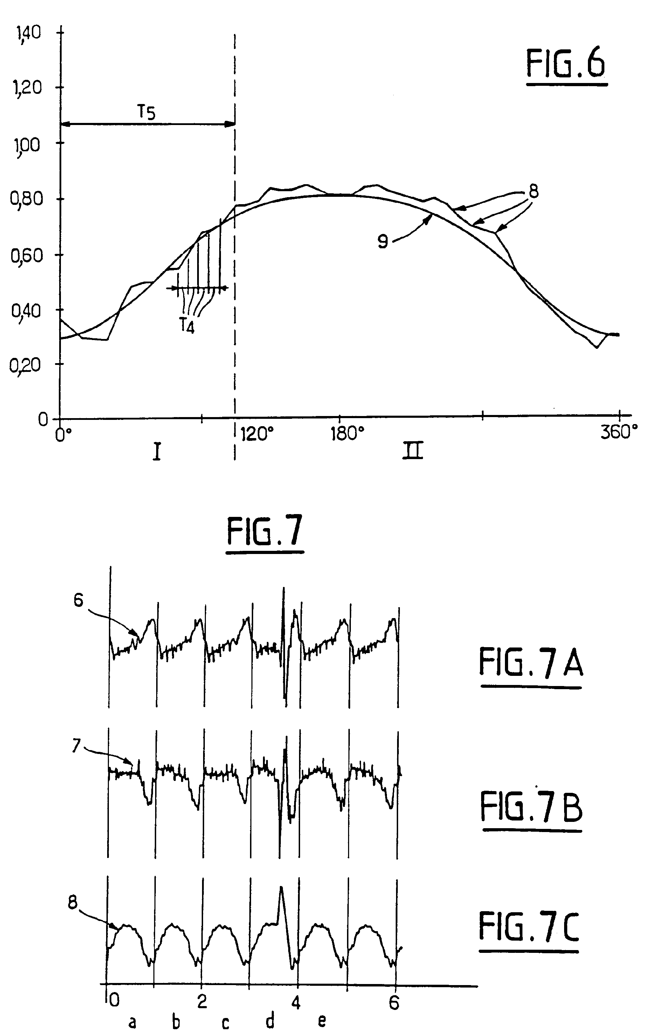

Sur la figure 6 est représenté un exemple de l'allure des variations de l'intensité absorbée par le moteur d'une pompe cardiaque à piston rotatif.

L'axe des abscisses représente le temps ou la position angulaire d'un rotor de la prothèse, quand celui-ci est entraîné à vitesse constante.

Cet axe est gradué de 0 à 360 degrés, ce qui correspond à un tour du rotor de la prothèse et à une période de variation du débit sanguin véhiculé par la prothèse.

L'axe des ordonnées représente l'intensité absorbée par le moteur.

La courbe de la figure 6 représente les valeurs captées 8 de l'intensité absorbée dans un cas de fonctionnement normal de la prothèse cardiaque.

Ces valeurs captées 8 ont été enregistrées pendant une période T.

Cette période T est divisée en deux phases I et II.

Dans le mode de mise en oeuvre d'un procédé selon l'invention représenté sur les figures 6 à 9, dans la première phase I de durée $T_5$ inférieure à T/2, on utilise lesdites valeurs captées 8 de l'intensité absorbée pour déterminer les conditions de fonctionnement de ladite prothèse qui influent normalement sur l'intensité absorbée par ledit moteur ; ces conditions sont par exemple la pression sanguine d'entrée de la pompe, la pression de sortie, le débit réel véhiculé par la prothèse.

En connaissant les paramètres physiques et géométriques de ladite prothèse qui conditionnent notamment l'allure de la courbe de variation du débit véhiculé théoriquement, ainsi que les caractéristiques dudit moteur, on peut alors prédéterminer les valeurs normales 9 approchées de l'intensité absorbée par ledit moteur qui doivent être observées dans la phase II.

Ces valeurs normales 9 forment la courbe 9 sur la figure 6.

Ces valeurs normales 9 prévisibles peuvent avantageusement être choisies sur une courbe représentant une fonction mathématique simple, par exemple une somme de fonctions trigonométriques dont les périodes sont des fractions de ladite période T de variation du débit.

On voit sur la figure 6 qu'à des intervalles de temps $T_4$ régulièrement espacés, on capte lesdites valeurs 8 dudit paramètre électrique de fonctionnement pour chaque valeur correspondante de ladite position de ladite prothèse, de sorte que on peut ensuite capter grâce audit capteur 11 des valeurs dudit paramètre physique de fonctionnement et simultanément on peut déterminer grâce auxdits moyens 10 la position de ladite prothèse, puis on peut calculer l'écart E entre ladite valeur captée dudit paramètre physique de fonctionnement et la valeur normale 9 dudit paramètre physique de ladite suite de valeurs normales correspondant à ladite position de ladite prothèse déterminée par lesdits moyens 10, et si ledit écart E est supérieur audit seuil, on peut agir sur lesdits moyens d'alimentation 5 pour réduire la vitesse d'entraînement de ladite pompe cardiaque.

Sur les figures 7A, 7B, 7C sont dessinées trois courbes 6, 7 et 8 qui représentent les variations respectives de la pression sanguine à l'entrée de ladite prothèse, de la pression sanguine à la sortie de la prothèse, de l'intensité absorbée par ledit moteur entraînant ladite prothèse.

Ces graphes 7A, 7B, 7C ont la même échelle sur l'axe des abscisses, qui est gradué en nombre de tour du rotor de ladite prothèse, ou en nombre de période de variation du débit véhiculé.

Cinq périodes sont représentées qui sont repérées de a à e.

Sur les figures 7A et 7B, l'axe des ordonnées représente la variation de pression sanguine.

Sur la figure 7C, l'axe des ordonnées représente l'intensité absorbée par ledit moteur et est gradué en ampères.

Les périodes a, b, c et e sont représentatives d'un fonctionnement correct de ladite prothèse ; la période d montre une perturbation qui provoque une variation très rapide des pressions à l'entrée et à la sortie de ladite pompe et de l'intensité absorbée par ledit moteur.

La figure 8 représente à une échelle agrandie par rapport à la figure 7C, lesdites valeurs captées 8 de l'intensité absorbée par ledit moteur de ladite prothèse pendant lesdites périodes a à e de la figure 7C.

Dans la figure 8, les graphes desdites périodes a à e de la figure 7C sont superposés.

L'axe des abscisses représente le temps ou la position angulaire d'un rotor de la prothèse ; il est gradué de 0 à 360 degrés et représente un tour ou une période.

L'axe des ordonnées représente l'intensité et est gradué en ampères.

Un axe parallèle à l'axe des abscisses défini une valeur 30 de l'intensité qui pourrait être la valeur de réglage d'un dispositif connu de limitation d'intensité du courant absorbé par le moteur électrique ; on voit que cet axe coupe la courbe desdites valeurs captées 8 correspondant à la période d où se produit la perturbation, en un point 13, d'abscisse 14. On voit qu'un dispositif de limitation d'intensité de type connu ne permet pas, même en supposant une action très rapide, de modifier à temps une consigne de fonctionnement de la prothèse, une consigne de vitesse par exemple et ne permet donc pas une régulation efficace et fiable du débit de ladite prothèse cardiaque.

La figure 9 montre les opérations d'un mode de mise en oeuvre d'un procédé selon l'invention, qui permettent de détecter l'anomalie de fonctionnement de la prothèse.

L'axe des abscisses est utilisé avec les mêmes conventions que dans les figures 6 et 8.

Les courbes Ea, Eb, Ec, Ed, Ec sont respectivement relatives auxdites périodes a à e.

La courbe Ea est la représentation de l'évolution pendant la période a (par référence à la figure 7c) dudit écart E entre lesdites valeurs captées 8 et lesdites valeurs normales 9 telles que décrites sur la figure 6.

On voit que dans la première phase I de la période, ledit écart 19A reste très proche de la valeur nulle ; en effet, durant cette phase, comme décrit pour la figure 6, on a calculé ou déterminé les valeurs normales 9 par rapport aux valeurs captées 8.

Ledit écart Ea augmente sensiblement plus vite dans la portion de courbe situé dans la zone II correspondant à la deuxième phase d'un procédé selon l'invention, puisque lesdites valeurs normales ont été entièrement prédéterminées à la fin de ladite phase I, et ne seront modifiées qu'à la période suivante.

Dans l'exemple représenté sur la figure 9, lesdits écarts Ea à Ee ont été calculés comme la somme quadratique des différences entre lesdites valeurs captées 8 et lesdites valeurs normales 9 ; on a donné une valeur nulle auxdits écarts Ea à Ee au début de ladite période correspondante ; dans l'exemple représenté, ladite durée $T_5$ de la dite première phase I est ici sensiblement égale au premier tiers de la période ; la durée de ladite deuxième phase II est égale aux deux derniers tiers de ladite période.

Il est clair que l'on pourrait calculer lesdits écarts Ea à Ee en fonction des différences desdites valeurs captées 8 et desdites valeurs normales 9 par d'autres méthodes connues par exemple la somme desdites différences, ou la somme des valeurs absolues desdites différences, lesquelles sommes pouvant être éventuellement pondérées par des coefficients ou des séries de coefficients choisies en fonction des observations expérimentales.

Les courbes Eb à Ee sont des courbes équivalentes à ladite courbe Ea, mais pour les périodes respectives b à e telle que définies à la figure 7.

Sur la figure 9, on voit une droite 17 qui représente l'évolution linéaire dudit seuil S pendant une période, depuis une valeur initiale nulle en début de période, jusqu'à une valeur finale 24.

On voit que lesdites courbes desdits écarts Ea à Ec et Ee restent en dessous de ladite droite de seuil 17 ; en effet pendant lesdites périodes a à c et e, lesdites valeurs captées 8 dans ladite phase II s'écartent peu desdites valeurs normales 9 qui ont été déterminées dans lesdites phases I correspondantes.

Par contre, on voit que ladite courbe Ed, représentative de ladite période pendant laquelle apparaît une dépression ou perturbation, coupe ladite droite 17 en un point 22 d'abscisse 23, puis dépasse largement cette droite de seuil ; dans un procédé selon l'invention, on va agir dans ce cas dès l'instant 23 de la détection du dépassement de ladite droite de seuil par ledit écart, sur lesdits moyens d'alimentation 5 dudit moteur de ladite prothèse pour adapter le débit sanguin de ladite pompe cardiaque au retour veineux ; on va de préférence diminuer la vitesse de ladite pompe cardiaque avantageusement, on pourra agir suffisamment rapidement pour éviter l'apparition des perturbations ou dépressions telles que représentées sur la figure 7A.

Dans les exemples représentés sur les figures 6 à 9, on a capté lesdites valeurs 8 de l'intensité absorbée par ledit moteur, à des intervalles de temps (repère $T_4$ de la figure 6) réguliers, 48 fois par période par exemple.

Dans d'autres procédés selon l'invention, on va utiliser de la même manière un autre paramètre électrique tel que tension, puissance par exemple ; dans ce cas, ledit capteur d'intensité pourra être remplacé par un capteur adapté au paramètre électrique à surveiller.

A la figure 10 on a représenté des opérations d'un mode préférentiel de réalisation d'un procédé de régulation selon l'invention.

L'axe des abscisses représente le temps et est gradué en minutes et l'axe des ordonnées représente la vitesse de rotation et/ou d'entraînement de ladite prothèse cardiaque.

Sur la figure 10, on a représenté sous la forme d'une courbe v l'évolution en fonction du temps, pendant une durée de l'ordre de 180 mn, des besoins d'assistance cardiaque d'un malade, qui évoluent en fonction du métabolisme dudit malade.

On sait en effet qu'en fonction des variations du métabolisme d'un individu, le débit sanguin qui doit être véhiculé, subi des variations et par conséquent le débit sanguin d'assistance qui peut être en totalité ou partiellement assuré par une prothèse cardiaque varie également au cours du temps.

On voit sur cette figure que la vitesse d'entraînement d'une prothèse représentée par ladite courbe v, qui permet d'assurer le débit sanguin correspondant à une assistance cardiaque totale, augmente dans une première partie de ladite courbe v jusqu'à une valeur de vitesse d'entraînement théorique $V_1$ puis diminue jusqu'à atteindre une valeur $V_2$, qui correspond sensiblement au temps 90 mn sur l'échelle des abscisses, puis augmente légèrement et diminue ensuite jusqu'à une valeur $V_8$ qui correspond à un palier à partir du repère 180 mn sur l'axe des temps de 180 mn.

Sur la figure 10, on a représenté une deuxième courbe repérée v' qui représente l'allure de l'évolution en fonction du temps, de la vitesse d'entraînement d'une prothèse cardiaque selon un procédé de régulation conforme à l'invention. On voit, au voisinage de l'abscisse repéré 0 sur l'axe des temps, qu'une vitesse initiale $V_3$, qui peut être quelconque, est prévue pour l'entraînement de ladite prothèse cardiaque ; sur le chronogramme représenté à la figure 10, on suppose que conformément à l'invention on a prévu un dispositif de réglage d'une consigne de pourcentage d'assistance, qui dans l'exemple représenté sur la figure 10 a été réglé à une valeur de l'ordre de 80 % ; ainsi conformément à l'invention, dans une première partie de ladite courbe V', on augmente ladite vitesse d'entraînement de ladite prothèse cardiaque à partir de ladite valeur initiale $V_3$ par paliers successifs de durée $T_1$, qui est avantageusement telle que le rapport $T_1/T$ soit inférieur à 500 et de préférence inférieur à 50 ; on voit qu'on augmente ainsi successivement ladite vitesse d'entraînement de ladite prothèse cardiaque jusqu'à atteindre une vitesse $V_4$ qui correspond sensiblement à la vitesse d'entraînement de la prothèse cardiaque qui assure un débit sanguin correspondant à une assistance totale dudit malade.

Du fait que ledit débit véhiculé par ladite prothèse fonctionnant à ladite vitesse d'entraînement $V_4$ assure une assistance totale, un très légèrement accroissement de ladite vitesse provoquera un dépassement dudit écart par rapport audit seuil conformément à l'invention, qui permettra ainsi de détecter que la vitesse courante, soit ladite vitesse V4, correspond à la vitesse maximale correspondant à ladite assistance totale ; de la sorte on peut alors réduire la vitesse d'entraînement de ladite pompe à une valeur $V'_4$ qui est obtenue de telle sorte que le rapport entre ladite vitesse $V'_4$ et ladite vitesse $V_4$ soit égal audit pourcentage d'assistance prédéterminé, soit dans l'exemple représenté à la figure 10 environ 80 % ; on voit que consécutivement à ladite phase d'augmentation progressive régulière de la vitesse qui a permis d'atteindre ladite vitesse $V_4$, ladite courbe V' représentative de la vitesse d'entraînement de ladite prothèse cardiaque redescend à ladite valeur $V'_4$ et peut rester à ladite valeur pendant un certain temps.

Conformément à l'invention, après une durée $T_2$ telle que le rapport $T_2/T$ soit supérieur à 1000 par exemple, on recommence une phase d'augmentation, de préférence régulière et progressive, de ladite vitesse d'entraînement de ladite prothèse cardiaque de façon à permettre de détecter à nouveau ladite vitesse maximale d'entraînement de ladite prothèse cardiaque correspondant à l'assistance cardiaque totale du malade, qui a évolué selon ladite courbe v.

On voit que dans le laps de temps situé entre les repères 60 et 90 de l'échelle des temps, on va donc augmenter ladite vitesse d'entraînement par palier dont la durée est sensiblement égale à ladite durée $T_1$ jusqu'à provoquer, du fait de l'accroissement de ladite vitesse, un dépassement dudit écart par rapport au seuil, lequel dépassement est caractéristique du fait que l'on a atteint une vitesse $V_5$ qui correspond à ce moment là, à la vitesse d'entraînement maximale qui correspond à l'assistance cardiaque totale ; on voit que dans un procédé selon l'invention, on réduit à nouveau ladite vitesse d'entraînement à une valeur $V'_5$ qui est obtenue de la même façon que précédemment par le rapport $V'_5/V_5$ sensiblement égal à 80 %, qui est le pourcentage prédéterminé d'assistance cardiaque.

On voit ensuite que conformément à l'invention, on effectue à nouveau après ledit intervalle de temps $T_2$, une nouvelle augmentation de préférence progressive et régulière de ladite vitesse d'entraînement, laquelle augmentation par paliers de durée sensiblement égale à ladite durée $T_1$ permet d'atteindre une vitesse $V_6$ qui provoque le dépassement dudit écart mesuré par rapport audit seuil, lequel dépassement est caractéristique de la vitesse correspondant à l'assistance totale, et que l'on réduit ensuite la vitesse d'entraînement de ladite pompe cardiaque comme représenté sur le graphe v' à une nouvelle vitesse $V'_6$ qui est obtenue par le rapport $V'_6/V_6$ sensiblement égal audit pourcentage d'assistance qui est dans cet exemple de 80 % ; et on voit que dans le laps de temps situé entre les repères 150 et 180 du graphe de la figure 10, on opère de même lorsqu'on détecte pendant ledit palier à ladite vitesse $V'_6$ un écart supérieur au seuil, qui correspond à une vitesse $V_7$ qui correspond elle-même à une assistance totale dudit malade, de sorte que l'on réduit la vitesse d'entraînement de ladite prothèse à une vitesse $V'_7$ qui est obtenue par rapport à ladite vitesse $V_7$ en multipliant ladite vitesse $V_7$ par ledit pourcentage d'assistance prédéterminé.

Les procédés et dispositifs de régulation selon l'invention peuvent avantageusement être appliqués à une prothèse telle que présentée dans les documents :

International Society for Artificial Organs, International workshop on Rotary Blood pumps OBERTAUERN (Austria) DEC 1-3, 1988 pages 24 à 27 et pages 28 à 30 (Proceedings Edited by H. Thomas, H. Schima - Bioengineering Laboratory - Ludwig Boltzmann Institut - 2nd Dept. of Surgery- University of Vienna).

## Revendications

1. Procédé de régulation d'une prothèse cardiaque qui comporte une pompe (3) volumétrique assurant un débit périodique de période (T) et qui comporte un actionneur électrique (18) d'entraînement de ladite pompe, lequel actionneur est alimenté par des moyens d'alimentation électrique (5), caractérisé en ce qu'il comporte les opérations suivantes:

a - on procure au moins un capteur (11) de mesure d'un paramètre physique de fonctionnement de ladite prothèse dont la bande passante s'étend au-delà de la fréquence (F) correspondant à ladite période (T) et on procure des moyens (10) de détermination de la position de ladite prothèse,

b - on détermine au moins une suite de valeurs normales (9) dudit paramètre physique de fonctionnement de ladite prothèse et une suite de positions (12) de ladite prothèse correspondant respectivement auxdites valeurs normales dudit paramètre physique, laquelle suite de valeurs normales (9) dudit paramètre physique doit être observée pendant ladite période (T),

    c - on détermine au moins un seuil (S),

        puis lors du fonctionnement de ladite prothèse, pendant chacune desdites périodes, à des intervalles de temps ($T_4$) de valeur inférieure à ladite période (T), et de préférence à des intervalles de temps ($T_4$) régulièrement espacés et tels que le rapport $T/T_4$ soit supérieur à 10,

    d - on capte grâce audit capteur (11) des valeurs dudit paramètre physique de fonctionnement et simultanément on détermine grâce auxdits moyens (10) la position de ladite prothèse,

    e - on calcule un écart (E) entre ladite valeur captée dudit paramètre physique de fonctionnement et la valeur normale (9) dudit paramètre physique de ladite suite de valeurs normales correspondant à ladite position de ladite prothèse déterminée par lesdits moyens (10),

    f - si ledit écart (E) est supérieur audit seuil, on agit sur lesdits moyens d'alimentation (5) pour réduire la vitesse d'entraînement de ladite pompe cardiaque.

**2.** Procédé selon la revendication 1 caractérisé en ce que lorsqu'on modifie la vitesse d'entraînement de ladite pompe, on initialise des moyens de temporisation de durée ($T_1$) tels que le rapport $T_1/T$ soit inférieur à 500 et de préférence inférieur à 50, et lorsque ladite durée ($T_1$) est écoulée, on agit sur lesdits moyens d'alimentation pour augmenter la vitesse d'entraînement de ladite pompe cardiaque.

**3.** Procédé selon l'une quelconque des revendications 1 à 2 caractérisé en ce qu'à des intervalles de temps ($T_2$) tels que le rapport $T_2/T$ soit supérieur à 1000, on agit sur lesdits moyens d'alimentation pour augmenter, de préférence progressivement et régulièrement, la vitesse d'entraînement de ladite pompe cardiaque jusqu'à ce que ledit écart selon ladite opération f soit supérieur audit seuil (S), de sorte que l'on détermine ainsi une vitesse maximale d'entraînement de ladite pompe, qui correspond à une assistance totale d'un malade.

**4.** Procédé selon la revendication 3 caractérisé en ce qu'on procure en outre des moyens de réglage d'une consigne de pourcentage d'assistance d'un malade, et en ce que consécutivement à la détection de ladite vitesse maximale d'entraînement de ladite pompe correspondant à une assistance totale, on réduit ladite vitesse d'entraînement de ladite pompe à une valeur sensiblement égale au produit de ladite vitesse maximale par ledit pourcentage d'assistance.

**5.** Procédé selon l'une quelconque des revendications 1 à 4 caractérisé en ce que ledit capteur (11) de mesure d'un paramètre physique de fonctionnement est un capteur de la composante fluctuante de la pression d'un fluide intervenant dans le fonctionnement de ladite prothèse cardiaque, lequel capteur est très peu sensible aux accélérations, et en ce que, préalablement à ladite opération b,

    - on capte les positions (12) de ladite pompe et simultanément on capte les amplitudes de la composante fluctuante de ladite pression dudit fluide intervenant dans le fonctionnement de ladite prothèse cardiaque correspondante, pendant une période (T) au moins,

    - on calcule des valeurs normales de la composante fluctuante de ladite pression dudit fluide intervenant dans le fonctionnement de ladite prothèse cardiaque correspondant auxdites positions (12) captées, lesquelles valeurs sont normalement très proches desdites amplitudes de la composante fluctuante de la pression dudit fluide intervenant dans le fonctionnement de ladite prothèse cardiaque, et sont situées sur une courbe (7) qui est la représentation graphique d'une fonction constituée par une somme de fonctions trigonométriques dont les périodes sont des fractions de ladite période (T).

**6.** Procédé selon la revendication 5 caractérisé en ce que ladite pression dudit fluide intervenant dans le fonctionnement de ladite prothèse est la pression sanguine à l'entrée de ladite pompe, et en ce que ladite pompe cardiaque est une pompe cardiaque rotative et lesdites positions sont les position angulaires d'un rotor de ladite pompe et/ou du moteur rotatif constituant ledit actionneur, et en ce que ladite suite de valeurs normales (9) de la composante fluctuante de la pression d'entrée ainsi que ledit seuil, sont enregistrés dans au moins une mémoire (16).

**7.** Procédé selon l'un quelconque des revendications 1 à 4 caractérisé en ce que ledit capteur (11) de mesure d'un paramètre physique de fonctionnement est un capteur d'un paramètre électrique du fonctionnement dudit actionneur, de préférence un capteur de l'intensité absorbée par ledit actionneur et en ce que dans ladite opération d, en début de ladite période, et pendant une durée ($T_5$) inférieure à la moitié de ladite période,

    - on utilise les valeurs captées (8) dudit paramètre électrique pour déterminer les conditions de fonctionnement de ladite pompe cardiaque,

- on prédétermine lesdites valeurs normales (9) dudit paramètre électrique qui doivent être observées dans la période courante, après ledit début de période de ladite durée $(T_5)$, en fonction desdites conditions de fonctionnement et desdites valeurs captées (8).

8. Procédé selon la revendication 7 caractérisé en ce que :
- on calcule ledit écart (E) comme étant la somme quadratique des écarts entre lesdites valeurs captées (8) depuis le début de ladite période et lesdites valeurs normales (9) dudit paramètre électrique,
- on donne une valeur nulle au début de chaque période audit écart (E) et audit seuil (S),
- on donne audit seuil (S) une valeur qui varie linéairement en fonction du temps écoulé depuis le début de ladite période.

9. Dispositif de régulation d'une prothèse cardiaque qui comporte une pompe (3) volumétrique assurant un débit périodique de période (T) et qui comporte un actionneur électrique (18) d'entraînement de ladite pompe, lequel actionneur est alimenté par des moyens d'alimentation électrique (5), caractérisé en ce qu'il comporte en outre au moins un capteur (11) de mesure d'un paramètre physique de fonctionnement de ladite prothèse dont la bande passante s'étend au-delà de la fréquence (F) correspondant à ladite période (T), et en ce qu'il comprend des moyens (10) de détermination de la position de ladite prothèse, au moins une mémoire (16) dans laquelle peuvent être stockées des valeurs normales dudit paramètre physique, au moins une unité centrale (15) qui reçoit des signaux issus dudit capteur (11) et desdits moyens (10), laquelle unité centrale peut commander lesdits moyens d'alimentation (5) pour faire varier la vitesse dudit actionneur et/ou de ladite pompe.

10. Dispositif selon la revendication 9 caractérisé en ce que ladite pompe est une pompe rotative et ledit actionneur est un moteur synchrone à aimants permanents, et lesdits moyens de détection de la position de ladite prothèse sont des moyens de sonde à effet Hall qui sont sensibles au champ électromagnétique rayonné par ledit moteur.

11. Dispositif selon l'une quelconque des revendications 9 à 10 caractérisé en ce que ladite pompe est raccordée à son entrée à un site d'implantation (1) et à sa sortie au réseau artériel par des canules (2) et ladite bande passante s'étend au-delà de 10 fois et de préférence au-delà de 100 fois ladite fréquence (F).

12. Dispositif selon l'une quelconque des revendications 9 à 11 caractérisé en ce que ledit capteur (11) est un capteur de pression sanguine qui est situé entre ledit site d'implantation et ladite entrée de ladite pompe, lequel capteur est très peu sensible aux accélérations, et en ce que des valeurs normales de la composante fluctuante de la pression sanguine à l'entrée de ladite pompe sont stockées dans ladite mémoire (16).

13. Dispositif selon l'une quelconque des revendications 9 à 11 caractérisé en ce que ledit capteur (11) est un capteur d'un paramètre électrique dudit actionneur, de préférence un capteur de l'intensité absorbée par ledit actionneur.

14. Dispositif selon l'une quelconque des revendications 9 à 13 caractérisé en ce qu'il comporte en outre des moyens (31) de réglage d'une consigne de pourcentage d'assistance d'un malade.

## Claims

1. Method of regulating a heart prosthesis which includes a positive displacement pump (3) providing a periodic flow of period (T) and which includes an electrical actuator (18) driving said pump, said actuator being powered by electrical power supply means (5), characterized in that it comprises the following operations:

a - at least one sensor (11) is provided for measuring a physical operating parameter of said prosthesis and having a passband extending beyond the frequency (F) corresponding to said period (T), and means (10) are provided for determining the position of said prosthesis;

b - at least one sequence of normal values (9) for said physical operating parameter of said prosthesis is determined together with a sequence of positions (12) of said prosthesis and corresponding to respective ones of said normal values of said physical parameter, which sequence of normal values (9) of said physical parameter should be complied with during said period (T);

c - at least one threshold (S) is determined;

then during operation of said prosthesis, during each of said periods, and at time intervals $(T_4)$ shorter than said period (T), and preferably at time intervals $(T_4)$ that are regularly spaced apart and that are such that the ratio $T/T_4$ is greater than 10;

d - values of said physical operating parameter are sensed by means of said sensor (11) and simultaneously the position of said prosthesis is determined by said means (10);

e - a difference (E) is calculated between said sensed value of said physical operating parameter and the normal value (9) of said physical parameter in said sequence of normal values and corresponding to said position of said prosthesis as determined by said means (10); and

f - if said difference (E) is greater than said threshold, action is taken on said power supply means (5) to reduce the drive speed of said heart pump.

2. Method according to claim 1, characterized in that when the drive speed of said pump is changed, timing means of duration $(T_1)$ are initialized where the ratio $T_1/T$ is less than 500 and is preferably less than 50, and when said time $(T_1)$ has elapsed, action is taken on said power supply means to increase the drive speed of said heart pump.

3. Method according to any one of claims 1 to 2, characterized in that action is taken on said power supply means at time intervals $(T_2)$ such that the ratio $T_2/T$ is greater than 1000, to increase, preferably progressively and regularly, the drive speed of said heart pump until said difference obtained in said operation f is greater than said threshold (S), thereby determining a maximum drive speed for said pump which corresponds to providing total assistance to a patient.

4. Method according to claim 3, characterized in that means are also provided for adjusting a reference value of percentage assistance for a patient, and in that on detecting said maximum drive speed of said pump corresponding to total assistance, said drive speed of said pump is reduced to a value substantially equal to the product of said maximum speed multiplied by said assistance percentage.

5. Method according to any one of claims 1 to 4, characterized in that said sensor (11) for measuring a physical operating parameter is responsive to the fluctuating component of the pressure of a fluid involved in the operation of said heart prosthesis, which sensor is very insensitive to acceleration, and in that prior to said operation b:

   - the positions (12) of said pump are sensed over at least one period (T) and simultaneously the amplitudes are sensed of the fluctuating component of said pressure of said fluid involved in the operation of said corresponding heart prosthesis; and

   - normal values of the fluctuating component of said pressure of said fluid involved in the operation of said heart prosthesis corresponding to said sensed positions (12) are calculated, which values are normally very close to said amplitudes of the fluctuating component of the pressure of said fluid involved in the operation of said heart prosthesis, and lie on a curve (7) which is the graphical representation of a function constituted by a sum of trigonometrical functions whose periods are fractions of said period (T).

6. Method according to claim 5, characterized in that said pressure of said fluid involved in the operation of said prosthesis is the blood pressure at the inlet to said pump, and in that said heart pump is a rotary heart pump and said positions are angular positions of a rotor of said pump and/or of the rotary motor constituting said actuator, and in that said sequence of normal values (9) of the fluctuating component of the inlet pressure and also said threshold are recorded in at least one memory (16).

7. Method according to any one of claims 1 to 4, characterized in that said sensor (11) for measuring a physical operating parameter is a sensor for an electrical parameter of the operation of said actuator, preferably a sensor responsive to the current taken by said actuator, and in that in said operation d at the beginning of said period, and throughout a duration $(T_5)$ less than half of said period:

   - the sensed values (8) of said electrical parameter are used to determine the operating conditions of said heart pump; and
   - said normal values (9) of said electrical parameter that ought to be observed during the present period are predetermined after said beginning of the period of said duration $(T_5)$, as a function of said operating conditions and of said sensed values (8).

8. Method according to claim 7, characterized in that:

   - said difference (E) is calculated as being the sum of the squares of the differences between said sensed values (8) from the beginning of said period and said normal values (9) of said electrical parameter;
   - a zero value is given at the beginning of each period to said difference (E) and to said threshold (S); and
   - said threshold (S) is given a value that varies linearly as a function of the time that has elapsed since the beginning of said period.

9. Apparatus for regulating a heart prosthesis which includes a positive displacement pump (3) providing a periodic flow of period (T) and which includes an electrical actuator (18) driving said pump, which actuator is powered by electrical power supply means (5), characterized in that it further includes at least one sensor (11) for measuring a physical operating parameter of said prosthesis and having a passband that extends beyond the frequency (F) corresponding to said period (T), and in that it comprises means (10) for determining the position of said prosthesis, at least one memory (16) in which normal values of said physical parameter can be stored, and at least one central unit (15) which receives the signals from said sensor (11) and from said means (10), which central unit is capable of controlling said power supply means (5) to vary the speed of said actuator and/or of said pump.

10. Apparatus according to claim 9, characterized in that said pump is a rotary pump and said actuator is a synchronous motor having permanent magnets, and said means for detecting the position of said prosthesis are Hall effect probe means responsive to the electromagnetic field radiated by said motor.

11. Apparatus according to any one of claims 9 to 10, characterized in that said pump has its inlet connected to an implantation site (1) and has its outlet connected to the arterial network via cannulas (2) and said passband extends to beyond ten times and preferably to beyond 100 times said frequency (F).

12. Apparatus according to any one of claims 9 to 11, characterized in that said sensor (11) is a blood pressure sensor situated between said implantation site and said inlet to said pump, which sensor is highly insensitive to acceleration, and in that normal values of the fluctuating components of the blood pressure at the inlet to said pump are stored in said memory (16).

13. Apparatus according to any one of claims 9 to 11, characterized in that said said sensor (11) is responsive to an electrical parameter of said actuator, and is preferably responsive to the current taken by said actuator.

14. Apparatus according to any one of claims 9 to 13, characterized in that it further includes means (31) for adjusting a reference value of assistance percentage for a patient.

**Patentansprüche**

1. Verfahren zur Regelung einer Herzprothese, die eine volumetrische Pumpe (3), die einen periodischen Durchsatz mit einer Periode (T) gewährleistet, und einen elektrischen Antrieb (18) zum Antreiben der Pumpe aufweist, wobei der Antrieb von einer elektrischen Stromversorgungseinrichtung (5) gespeist wird,
dadurch **gekenzeichnet**, daß es folgende Schritte umfaßt:
   a) Vorsehen mindestens eines Meßfühlers (11) zur Messung eines physikalischen Betriebsparameters der Herzprothese, dessen durchgelassener Frequenzbereich sich oberhalb der der Periode T entsprechenden Frequenz (F) erstreckt, und einer Einrichtung (10) zur Ermittlung der Stellung der Herzprothese,
   b) Bestimmung mindestens einer Folge von normalen Werten (9) des physikalischen Betriebsparameters der Herzpumpe und einer Folge von Stellungen (12) der Herzprothese, die den normalen Werten des physikalischen Betriebsparameters jeweils entsprechen, wobei die Folge von normalen Werten (9) des physikalischen Betriebsparameters während der Periode (T) ermittelt werden muß,
   c) Bestimmung mindestens eines Schwellenwertes (S),
   d) anschließendes Erfassen der Werte des physikalischen Betriebsparameters mit dem Meßfühler (11) und gleichzeitige Ermittlung der Stellung der Herzprothese mit der Einrichtung (10) im Betrieb der Herzprothese während jeder Zeitperiode in Zeitintervallen (T4), die kürzer sind als die Periode T, und vorzugsweise in solchen regelmäßigen Zeitintervallen (T4), daß das Verhältnis T/T4 größer als 10 ist,
   e) Berechnung einer Abweichung (E) zwischen dem erfaßten Wert des physikalischen Betriebsparameters und dem normalen Wert (9) des physikalischen Betriebsparameters der Folge von normalen Werten, welcher der durch die Einrichtung (10) ermittelten Stellung der Herzprothese entspricht,
   und
   f) Einwirken auf die Stromversorgungseinrichtung (5) zur Verringerung der Geschwindigkeit des Antriebs der Herzprothese, wenn die Abweichung (E) oberhalb des Schwellenwerts liegt.

2. Verfahren nach Anspruch 1,
dadurch gekennzeichnet, daß
dann, wenn die Geschwindigkeit des Antriebs der Pumpe geändert wird, eine Einrichtung zur Verzögerung um die Dauer T1 initialisiert wird, die so arbeitet, daß das Verhältnis T1/T kleiner als 500 und vorzugsweise kleiner als 50 ist, und nach Ablauf der Dauer T1 auf die Stromversorgungseinrichtung eingewirkt wird, um die Geschwindigkeit des Antriebs der Herzprothese zu erhöhen.

3. Verfahren nach Anspruch 1 oder 2, gekennzeichnet durch Einwirkung auf die Stromversorgungseinrichtung in Zeitintervallen (T2) die so sind, daß das Verhältnis T2/T1 größer als 1000 ist, zur vorzugsweise progressiven und regelmäßigen Erhöhung der Geschwindigkeit des Antriebs der Herzprothese, bis die Abweichung gemäß Schritt f) oberhalb des Schwellenwerts (S) liegt, um so eine maximale Geschwindigkeit des Antriebs der Pumpe zu bestimmen, die einer totalen Unterstützung eines Kranken entspricht.

4. Verfahren nach Anspruch 3, dadurch gekennzeichnet, daß ferner eine Einrichtung zur Einstellung eines Sollwerts der prozentualen Unterstützung eines Kranken vorgesehen wird und im Anschluß an die Erfassung der einer totalen Unterstützung entsprechenden maximalen Geschwindigkeit des Antriebs der Pumpe die Geschwindigkeit des Antriebs der Pumpe auf einen Wert verringert wird, der im wesentlichen gleich dem Produkt aus der maximalen Geschwindigkeit und der prozentualen Unterstützung ist.

5. Verfahren nach einem der Ansprüche 1 bis 4, dadurch gekennzeichnet, daß der Meßfühler (11) für einen physikalischen Betriebsparameter ein Fühler für die fluktuierende Komponente des Drucks eine Fluids ist, das im Betrieb der Herzprothese vorliegt, wobei der Fühler sehr geringe Empfindlichkeit gegenüber Beschleunigungen aufweist, sowie dadurch, daß, vor Schritt b),
   - die Stellungen (12) der Pumpe und gleichzeitig die Amplituden der fluktuierenden Komponente des Drucks des Fluids, das im Betrieb der entsprechenden Herzprothese vorliegt, während mindestens einer Periode T erfaßt werden, und
   - die normalen Werte der fluktuierenden Komponente des Drucks des Fluids, das im Betrieb der Herzprothese vorliegt, die den erfaßten Stellungen (12) entsprechen, wobei diese Werte normalerweise sehr nahe an den Amplituden der fluktuierenden Komponente des Drucks des Fluids, die im Betrieb der Herzprothese auftritt, liegen, und ferner auf einer Kurve (7) liegen, welche die graphische Darstellung einer Funktion ist, die aus einer Summe trigonometrischer Funktionen besteht, deren Perioden Bruchteile der Periode T sind.

6. Verfahren nach Anspruch 5, dadurch gekennzeichnet, daß der Druck des Fluids, das im Betrieb der Herzprothese vorliegt, der Blutdruck am Eingang der Pumpe ist, die Herzpumpe eine rotierende Herzpumpe ist, die Stellungen die Winkelstellungen eines Rotors der Pumpe und/oder des sich drehenden Motors sind, der den Antrieb darstellt, und die Folge von normalen Werten (9) der fluktuierenden Komponente des Eingangsdrucks sowie der Schwellenwert in mindestens einem Speicher (16) gespeichert werden.

7. Verfahren nach einem der Ansprüche 1 bis 4, dadurch gekennzeichnet, daß der Meßfühler (11) zur Messung eines physikalischen Betriebsparameters ein Fühler für einen elektrischen Betriebsparameter des Antriebs ist, vorzugsweise ein Fühler für die vom Antrieb aufgenommene Stromstärke, und in Schritt d) zu Beginn der Periode und während einer Dauer (T5), die kleiner ist als die Hälfte der Periode,
   - die erfaßten Werte (8) des elektrischen Betriebsparameters zur Ermittlung der Betriebsbedingungen der Herzpumpe verwendet werden, und
   - die normalen Werte (9) des elektrischen Betriebsparameters, die in der laufenden Periode ermittelt werden müssen, nach dem Beginn der Periode der Dauer T5 in Abhängigkeit von den Betriebsbedingungen und den erfaßten Werten (8) vorherbestimmt werden.

8. Verfahren nach Anspruch 7, dadurch gekennzeichnet, daß
   - die Abweichung (E) als Summe der Quadrate der Abweichungen zwischen den seit dem Beginn der Periode erfaßten Werten und den normalen Werten (9) des elektrischen Parameters berechnet werden,
   - die Abweichung (E) und der Schwellenwert (S) am Beginn jeder Periode gleich Null gesetzt werden, und
   - der Schwellenwert (S) einen Wert erhält, der sich in Abhängigkeit von der seit dem Beginn der Periode verstrichenen Zeit linear ändert.

9. Vorrichtung zur Regelung einer Herzprothese, die eine volumetrische Pumpe (3), die einen periodischen Durchsatz mit einer Periode (T) gewährleistet und einen elektrischen Antrieb (18) zum Antreiben der Pumpe aufweist, wobei der Antrieb von einer elektrischen Stromversorgungseinrichtung gespeist wird, dadurch gekennzeichnet, daß sie ferner mindestens einen Meßfühler (11) zur Messung eines physikalischen Betriebsparameters der Herzpumpe, dessen durchgelassener Frequenzbereich sich oberhalb der der Periode (T) entsprechenden Frequenz (F) erstreckt, eine Einrichtung (10) zur Ermittlung der Stellung der Herzpumpe, mindestens einen Speicher (16), in dem normale Werte des physikalischen Betriebsparameters gespeichert werden können, sowie mindestens eine Zentraleinheit (15) aufweist, die Signale vom Meßfühler (11) und der Einrichtung (10) empfängt und die Stromversorgungseinrichtung (5) steuern kann, um die Geschwindigkeit des Antriebs und/oder der Pumpe zu ändern.

10. Vorrichtung nach Anspruch 9, dadurch gekennzeichnet, daß die Pumpe eine rotierende Pumpe ist, der Antrieb eine Synchronmotor mit Permanentmagneten ist und die Einrichtung (10) zur Ermittlung der Stellung der Herzprothese eine Hallsondeneinrichtung ist, die auf das elektromagnetische Streufeld des Motors anspricht.

11. Vorrichtung nach Anspruch 9 oder 10, dadurch gekennzeichnet, daß die Pumpe über Kanülen (2) mit ihrem Eingang an eine Implantationsstelle (1) und mit ihrem Ausgang an das Arteriensystem angeschlossen ist und der durchgelassene Frequenzbereich sich jenseits der zehnfachen und vorzugsweise jenseits der hundertfachen Frequenz F erstreckt.

12. Vorrichtung nach einem der Ansprüche 9 bis 11, dadurch gekennzeichnet, daß der Meßfühler (11) ein Blutdruckfühler ist, der sich zwischen der Implantationsstelle und dem Eingang der Pumpe befindet und sehr geringe Empfindlichkeit gegenüber Beschleunigungen aufweist, und normale Werte der fluktuierenden Komponente des Blutdrucks am Eingang der Pumpe im Speicher (16) gespeichert werden.

13. Vorrichtung nach einem der Ansprüche 9 bis 11, dadurch gekennzeichnet, daß der Meßfühler (11) ein Fühler für einen elektrischen Parameter des Antriebs ist, vorzugsweise ein Fühler für die vom Antrieb aufgenommene Stromstärke.

14. Vorrichtung nach einem der Ansprüche 9 bis 13, dadurch gekennzeichnet, daß sie ferner eine Einrichtung (31) zur Einstellung eines Sollwerts für die prozentuale Unterstützung

eines Kranken aufweist.

# FIG.1

# FIG.2

EP 0 513 176 B1

FIG.3

FIG.4

18

# FIG.5

EP 0 513 176 B1

FIG.6

FIG.7

FIG.7A

FIG.7B

FIG.7C

EP 0 513 176 B1

FIG.8

FIG.9

# FIG.10

$$\frac{V'_4}{V_4} = \frac{80}{100} = \frac{V'_5}{V_5} = \frac{V'_6}{V_6} = \frac{V'_7}{V_7}$$

EP 0 513 176 B1